# EUROPEAN PATENT SPECIFICATION

(11) **EP 4 074 241 B1**
(45) Date of publication and mention of the grant of the patent: **18.02.2026**
(21) Application number: 22165224.1
(22) Date of filing: 29.03.2022
(51) Int. Cl.: A61B 1/00

(54) **ENDOSCOPE SYSTEM**
ENDOSKOPSYSTEM
SYSTÈME D'ENDOSCOPE

(30) Priority: 14.04.2021 JP 2021068568
(43) Date of publication of application: 19.10.2022
(73) Proprietor: i-PRO Co., Ltd., Tokyo 108-6014 (JP)
(72) Inventor: KOGANE, Haruo, Fukuoka, 812-8531 (JP)
(74) Representative: Eisenführ Speiser

(56) References cited:
- EP-A1- 2 912 987
- WO-A1-2018/005842
- JP-A- 2007 319 622
- JP-A- 2019 097 661

## Description

### TECHNICAL FIELD

The present disclosure relates to an endoscope system.

### BACKGROUND ART

A method for displaying guidance information using a graphical user interface during an medical procedure according to WO 2018/005842 A1 comprises displaying, in a first mode of the graphical user interface, first image data from a perspective corresponding to a distal end of an elongate device. The first image data includes a virtual roadmap. The method also comprises transitioning from the first mode of the graphical user interface to a second mode of the graphical user interface. The transition is based on an occurrence of a triggering condition. The method also comprises displaying, in the second mode of the graphical user interface, second image data from a perspective corresponding to the distal end of the elongate device. The second image data includes a target indicator corresponding to a target location and an alignment indicator corresponding to an expected location of the medical procedure at the target location.

EP 2 912 987 A1 relates to an insertion system, an insertion support device, an insertion support method and a program. An inserting state acquiring section acquires inserting state information of an inserting section that is to be inserted into an insertion subject, an insertion subject shape acquiring section acquires shape information of the insertion subject, and a positional relation calculating section is input the inserting state information and the insertion subject shape information and calculates a positional relation of the inserting section to the insertion subject. When an output section outputs the calculation result of the positional relation calculating section, a control section controls an output state of the calculation result in the output section.

In order to provide an endoscope navigation device for navigating procedures of a curvature operation and operations of forward movement/retreat movement and rotation of an insertion part by a computer, according to each portion of a lumen where an endoscope passes, in which, information related to endoscope insertion by navigation is accumulated in a database, and a program of navigation is updated by machine learning based on the database, and accuracy is enhanced, it is proposed in JP 2019-097661 A that an endoscope navigation device comprises an imaging device of an endoscope image, and a navigation function for presenting by an image or a voice, at least two of insertion procedures of an endoscope to a lumen of a body, simultaneously or parallel, in an endoscope device.

In order to provide an endoscope system, in which a desired curvature state can be set in short time, it is proposed in JP 2007-319622 A that on a tip side of an insert par, sensors to detect the curvature state of a curvature part are provided. A CPU determines relationship between a curvature state designated by a remote control and the curvature state detected by the sensors, and stores the information in an internal memory as precision data. In a designated curvature mode, the CPU uses the information to control a curvature drive part to set the curvature state close to the designated curvature state.

Patent Literature 1 discloses a blood vessel diameter measurement system, including: an endoscope including a tip provided with an image sensor that can be inserted into a blood vessel of a subject and can image the blood vessel; a drive device that moves the endoscope inserted into the blood vessel toward a proximal end side at a constant speed; and a calculation device that measures a blood vessel diameter of the blood vessel based on at least one pre-movement image captured before the endoscope is moved by the drive device, at least one post-movement image captured after the endoscope is moved by the drive device, and a traveling distance of the endoscope driven by the drive device.

### CITATION LIST

### PATENT LITERATURE

Patent Literature 1: JP-2020-185081-A

### SUMMARY OF INVENTION

According to a configuration of Patent Literature 1, it is premised that the images captured before and after the movement of the endoscope for measuring the blood vessel diameter are aligned in a vertical direction (in other words, an up-down direction) of the images. However, the blood vessel in the subject such as a human body is irregularly curved in some places, and when the endoscope is inserted in or pulled out from the blood vessel, the tip portion of the endoscope may rotate so that the up-down direction may deviate. In this case, users such as doctors who visually check the images captured by the endoscope cannot accurately understand conditions of an observed site in the subject, and it may be difficult to implement appropriate medical practice.

The present disclosure provides an endoscope system that efficiently detects presence or absence of deviation in an up-down direction of a tip portion of an endoscope and supports implementation of appropriate medical practice.

An endoscope system according to the present invention is defined in claim 1.

According to the present disclosure, it is possible to efficiently detect the presence or absence of deviation in the up-down direction of the tip portion of the endoscope and support the implementation of appropriate medical practice.

### BRIEF DESCRIPTION OF DRAWINGS

Fig. 1 is a diagram illustrating an example of a use case of an endoscope system according to a first embodiment;
Fig. 2 is a schematic diagram schematically illustrating a hardware configuration of the endoscope system illustrated in Fig. 1;
Fig. 3 is a functional block diagram functionally illustrating a configuration of the endoscope system illustrated in Fig. 2;
Fig. 4 is a schematic diagram schematically illustrating a hardware configuration of a tip portion (camera head portion) of an endoscope illustrated in Fig. 2;
Fig. 5 is a functional block diagram illustrating processing by a processor of a camera control unit illustrated in Fig. 4;
Fig. 6 is a flow chart illustrating a processing procedure for superimposing and displaying an indicator on an image;
Fig. 7A is a schematic diagram illustrating a first example of an image at each insertion position before the processing illustrated in Fig. 6 is executed;
Fig. 7B is a schematic diagram illustrating a second example of the image at each insertion position before the processing illustrated in Fig. 6 is executed;
Fig. 7C is a schematic diagram illustrating a third example of the image at each insertion position before the processing illustrated in Fig. 6 is executed;
Fig. 7D is a schematic diagram illustrating a fourth example of the image at each insertion position before the processing illustrated in Fig. 6 is executed;
Fig. 8A is a schematic diagram illustrating a first example of an image after the processing illustrated in Fig. 6 is executed on the image illustrated in Figs. 7A to 7D;
Fig. 8B is a schematic diagram illustrating a second example of the image after the processing illustrated in Fig. 6 is executed on the image illustrated in Figs. 7A to 7D;
Fig. 8C is a schematic diagram illustrating a third example of the image after the processing illustrated in Fig. 6 is executed on the image illustrated in Figs. 7A to 7D;
Fig. 9 is a schematic diagram illustrating a hardware configuration of a rotary drive device according to a second embodiment;
Fig. 10A is a schematic diagram illustrating a first example of a case where a transmission cable is rotated by the rotary drive device;
Fig. 10B is a schematic diagram illustrating a second example of the case where the transmission cable is rotated by the rotary drive device;
Fig. 11 is a flow chart illustrating a processing procedure for controlling rotation of the transmission cable around an axis; and
Fig. 12 is a graph illustrating a case where a rotational force of the transmission cable around the axis is controlled and converged to a predetermined value.

### DESCRIPTION OF EMBODIMENTS

Hereinafter, a plurality of embodiments in which the endoscope system according to the present disclosure is specifically disclosed will be described in detail with reference to the drawings as appropriate. However, unnecessarily detailed description may be omitted. For example, detailed description of a well-known matter or repeated description of substantially the same configuration may be omitted. This is to avoid unnecessary redundancy in the following description and to facilitate understanding for those skilled in the art. In addition, each of the accompanying drawings is referred to in accordance with a direction of the reference numerals. The accompanying drawings and the following description are provided for those skilled in the art to fully understand the present disclosure, and are not intended to limit the subject matter described in the claims.

For example, in the present disclosure, a medical endoscopic system (vascular endoscopic system, which will be described later) inserted into a blood vessel as an endoscopic system will be described as an example (a use case), but the present disclosure is not limited to this example. Observation sites in the subject include not only blood vessels but also digestive organs such as the stomach or large intestine, and the contents of the present disclosure can also be appropriately applied to an industrial endoscope system capable of observing pipes in a plant. The content of the present disclosure can be adopted for various applications as long as the content of the present disclosure can capture an image of a tubular subject.

**In** addition, the "unit" or the "device" in the embodiments is not limited to a physical configuration that is simply mechanically implemented by hardware, and includes a configuration of which a function is implemented by software such as a program. **In** addition, a function of one configuration may be implemented by two or more physical configurations, or functions of two or more configurations may be implemented by, for example, one physical configuration.

### (First Embodiment)

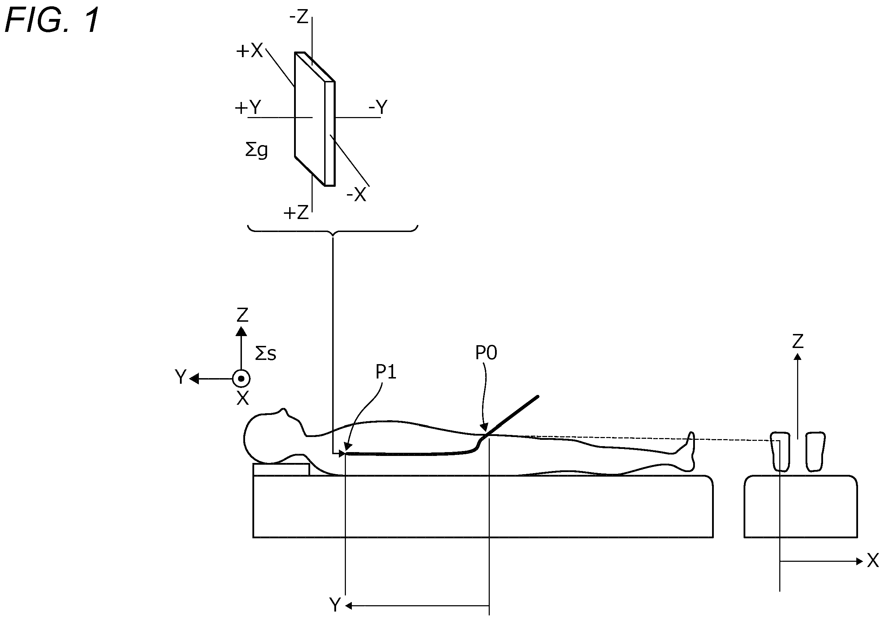

A first embodiment will be described with reference to Figs. 1 to 8.

### [Use Case of Endoscope System of Present Embodiment]

An example of a use case in which an endoscope system 1 of the present embodiment is used will be described with reference to Fig. 1. Fig. 1 is a diagram illustrating the example of the use case of the endoscope system 1 according to the first embodiment.

As shown in Fig. **1****,** the endoscope system 1 of the present embodiment is a dedicated medical device used during surgery or inspection, in which an endoscope camera 10 is attached to a tip portion of a transmission cable 2 (including a catheter and the like). The transmission cable 2 is provided with an outer peripheral surface covered with an insulating film, and connects from a proximal end portion to a tip portion of the endoscope camera 10 (an example of an endoscope). **In** the endoscope system **1,** the endoscope camera 10 connected to the transmission cable 2 is inserted into a subject of a human body, and a state of an observation site (for example, a blood vessel) in the subject is imaged (observed) by the endoscope camera 10.

The endoscopic camera 10 may be referred to as a so-called vascular endoscopic catheter. An outer diameter of the endoscope camera 10 is, for example, 1.8 mmΦ as a maximum outer diameter, but is not limited to this size. The endoscope camera 10 can be inserted in and pulled out from and can be freely moved forward and backward in, for example, a blood vessel in the subject along a guide wire (not shown) pre-inserted into the blood vessel in the subject.

Here, a direction in which the endoscope camera 10 is inserted toward the observation site in the subject is defined as a traveling direction, and oppositely, a direction in which the endoscope camera 10 is pulled out toward an outside of the subject is defined as an evacuation direction. That is, being inserted and pulled out or being freely moved forward and backward means that the endoscope camera 10 can be inserted toward or pulled out from an inside of the subject. The endoscope camera 10 can be smoothly inserted to the observation site by being guided by a guide wire pre-inserted to a site to be operated or inspected (for example, an affected area). In the endoscope system 1, the endoscope camera 10 may be interchangeably attached to a tip portion of a normal catheter. A catheter is, for example, a medical tube used for draining body fluids or injecting drug solutions. In addition to the endoscopic camera 10, a balloon, a stent, or the like may be interchangeably attached to the catheter.

In the present embodiment, a standard coordinate system Σs is set (see Fig. 1). In the standard coordinate system Σs, a Z direction is set along a direction of gravity, a Y direction is set along an axial direction of a trunk in a state where a patient is lying on an operating table, and an X direction is set along a left-right width direction of the trunk. That is, a Y-axis of the standard coordinate system Σs is in a direction along the above-mentioned traveling direction, and Fig. 1 shows an example of insertion into a femoral vein (inguinal region). In Fig. 1, a first insertion position at which insertion is started is indicated by a reference numeral "P0", and a second insertion position at which the endoscope camera 10 is further inserted in the subject and advanced further than the first insertion position P0 is indicated by a reference numeral "P1".

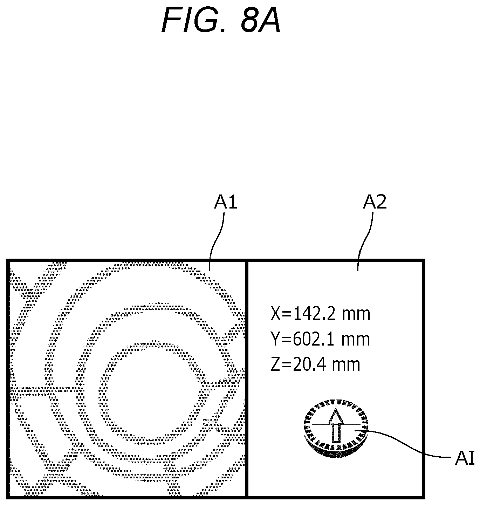

As will be described later, in the present embodiment, an acceleration sensor 17 (an example of a posture control sensor) is arranged on a camera head portion 11 of the endoscope camera 10, and an acceleration sensor coordinate system Σg is set for the acceleration sensor 17 (see Fig. 1). Since the acceleration sensor 17 can also detect a gravitational acceleration, an amount of deviation (deviation angle) between the acceleration sensor coordinate system Σg and the standard coordinate system Σs in the Z direction can be calculated. This calculation result means how much an image is rotated and deviated in the Z direction of the standard coordinate system Σs, in other words, in the up-down direction, and based on the calculation result, an indicator showing the deviation angle is superimposed and displayed on the image (see Figs. 8A to 8C).

### [Configuration of Endoscope System]

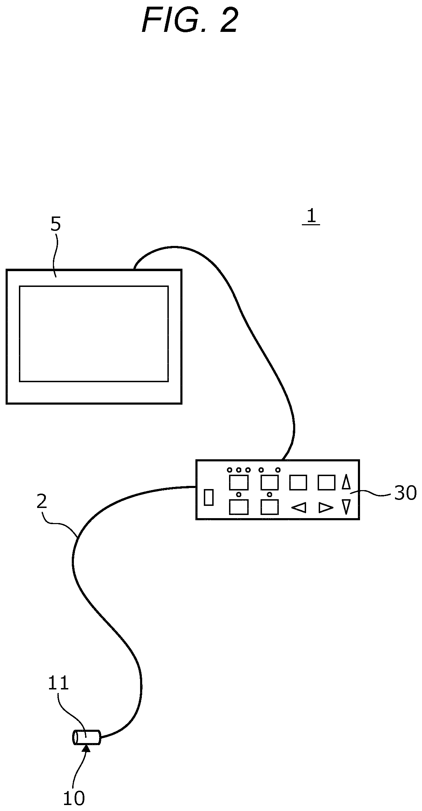

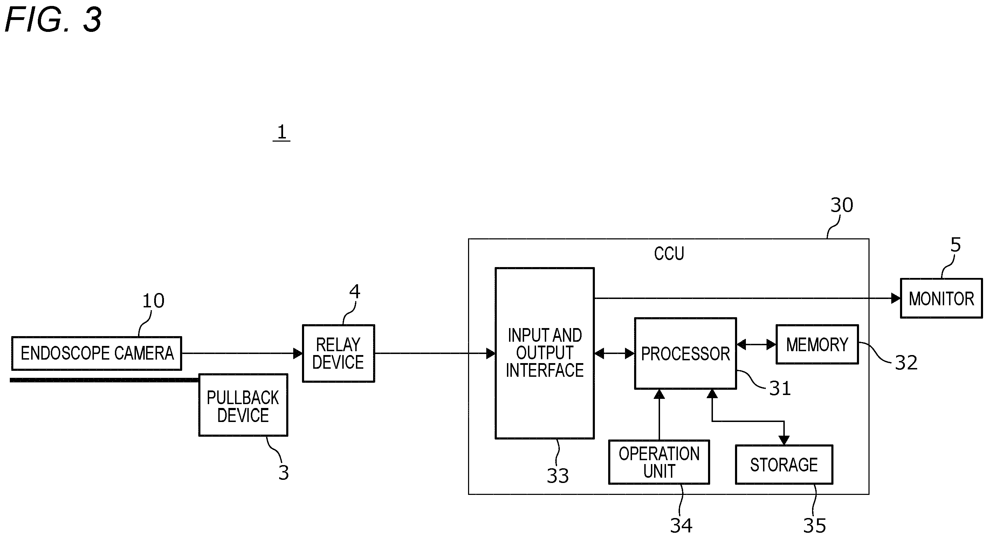

A hardware configuration of the endoscope system 1 will be described with reference to Figs. 2 and 3. Fig. 2 is a schematic diagram schematically illustrating the hardware configuration of the endoscope system 1 illustrated in Fig. 1. Fig. 3 is a functional block diagram functionally illustrating a configuration of the endoscope system 1 illustrated in Fig. 2. For convenience of explanation, a pullback device (see Fig. 3) and a relay device 4 (see Fig. 3) are not shown in Fig. 2.

As shown in Figs. 2 and 3, the endoscope system 1 includes the endoscope camera 10, an auto pullback device 3, a relay device 4, a camera control unit 30 (CCU, which is an example of a calculation device), and a monitor 5.

The endoscope camera 10 is, for example, a 480,000-pixel high-resolution camera including a tip portion (hereinafter also referred to as "camera head portion 11") provided with an image sensor 16 (an example of an image sensor, see below) capable of imaging a blood vessel. **In** the present embodiment, along with the image sensor 16, the acceleration sensor 17 (an example of the posture control sensor, see below) is also provided on the tip portion of the endoscope camera 10. When the endoscope camera 10 is inserted into, for example, a blood vessel in the subject, it is possible to image an inner wall of the blood vessel (hereinafter, also referred to as "blood vessel wall"). The number of pixels of 480,000 is merely an example, and the number of pixels is not limited to 480,000.

The auto pullback device 3 pulls back (in other words, tows) the endoscope camera 10 guided by the guide wire and inserted into the observation site (for example, the blood vessel) to a proximal end side at a pullback speed (for example, a constant speed). **In** this case, it is possible to comprehend that a length at which the auto pullback device 3 pulls back the transmission cable 2 (in other words, the endoscope camera 10) is substantially the same as a length at which the endoscope camera 10 inserted at a position near the affected area is pulled back toward the proximal end side. The endoscope system 1 images the blood vessel wall at equal intervals when the endoscope camera 10 is pulled back by the auto pullback device 3. The auto pullback device 3 outputs position information of the endoscope camera 10 and speed information of an imaging position to the relay device 4.

Here, the speed information of an imaging position of the endoscope camera 10 is a speed at which an imaging position of the endoscope camera 10 (that is, a position of the endoscope camera 10) moves to the proximal end side at a constant speed when the endoscope camera 10 is pulled back to the proximal end side, and is substantially the same as the pullback speed. The speed information of an imaging position of the endoscope camera 10 is set by the auto pullback device 3 by an operation of a user such as a doctor. A start position of imaging is determined after, for example, a user such as a doctor confirms the image of the observation site (for example, a blood vessel) of the subject captured by the endoscope system 1 on the monitor 5. The pullback speed may be constant or may be changed.

The relay device relays various signals transmitted between the endoscope camera 10 and the camera control unit 30. The various signals include, for example, various control signals for the camera control unit 30 to control the endoscope camera 10, and data signals (image data and the like) of the image captured by the endoscope camera 10. Further, in the present embodiment, the various signals include an acceleration signal detected by the acceleration sensor 17 of the endoscope camera 10.

The camera control unit 30 includes a processor 31, a memory 32, an input and output interface 33, an operation unit 34, and a storage 35.

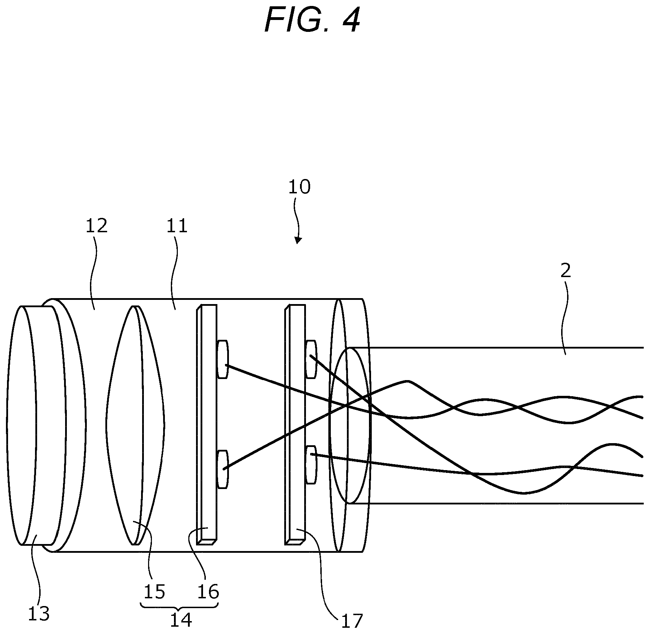

The camera control unit 30 is electrically connected to the endoscope camera 10 via the relay device 4, and controls an imaging operation by the endoscope camera 10 and generation of an image based on a signal of the image from the endoscope camera 10. In the present embodiment, the camera control unit 30 acquires an acceleration signal from the acceleration sensor 17 (see Fig. 4) of the endoscope camera 10, and calculates the amount of deviation (deviation angle) between the acceleration sensor coordinate system Σg and the standard coordinate system Σs in the Z direction based on an acquired result. The camera control unit 30 displays, on the monitor 5, an animation icon AI of a compass corresponding to the amount of deviation (see below, see Figs. 8A to 8C) with the animation icon AI superimposed on the image.

The camera control unit 30 includes at least an image input unit (not shown), an image processing unit (not shown), and an image output unit (not shown) as a part of functions thereof. These functions are implemented by, for example, being stored and retained in the memory 32 as a program and executed by the processor 31.

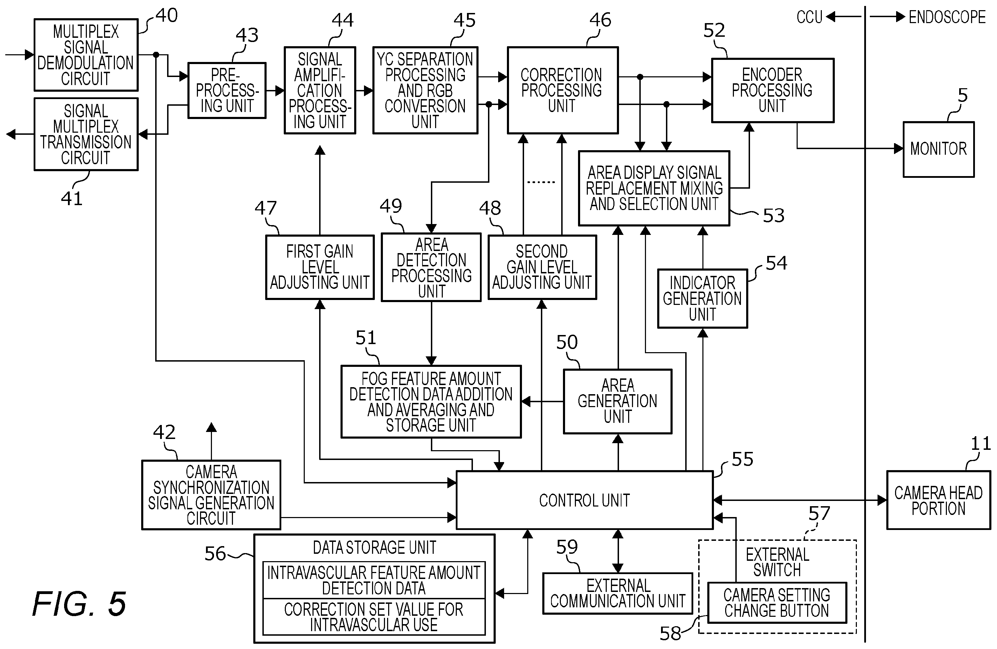

The image input unit inputs an image of the blood vessel wall or the like captured by the endoscope camera 10 via the input and output interface 33. The image processing unit performs a processing such as superimposing an indicator such as the animation icon AI of the compass on the input image of the blood vessel wall or the like. The image output unit outputs the image to the monitor 5 via the input and output interface 33. The functions such as the image input unit (not shown), the image processing unit (not shown), and the image output unit (not shown) are comprehensively implemented by each of functional blocks shown in Fig. 5, which will be described later.

The processor 31 of the camera control unit 30 implements various functions of the endoscope system 1 by executing programs stored in the memory 32 as described above. The processor 31 may be provided by, for example, a graphical processing unit (GPU) suitable for image processing. Instead of the GPU, the processor 31 may be configured by a dedicated electronic circuit designed by a micro processing unit (MPU), a central processing unit (CPU), an application specific integrated circuit (ASIC), or the like, or an electronic circuit designed to be reconfigurable by a field programmable gate array (FPGA) or the like.

The memory 32 is used as a working memory of the processor 31. In addition to the dedicated image input interface, the input and output interface 33 may be an interface using a high-definition multimedia interface (HDMI, registered trademark), a universal serial bus (USB) Type-C, or the like capable of transferring video data at a high speed.

The operation unit 34 receives an operation by the user such as a doctor. The operation unit 34 may be configured by one or a combination of a mouse, a keyboard, a touch pad, a touch panel, a microphone, or other input devices.

The storage 35 is a large-capacity storage device, and stores data of images of the blood vessel wall and the like captured by the endoscope camera 10. The storage 35 may be configured to include, for example, a secondary storage device (for example, a hard disk drive (HDD) or a solid state drive (SSD)), or a tertiary storage device (for example, an optical disk and an SD card).

The monitor 5 displays a measurement result of a blood vessel diameter output from the camera control unit 30 or the image of the blood vessel wall or the like. When displaying the image of the blood vessel wall, the monitor 5 can three-dimensionally display the image as a three-dimensional image of the blood vessel viewed from a direction desired by the user. The monitor 5 includes a display device such as a liquid crystal display (LCD), an organic electroluminescence (EL), or a cathode ray tube (CRT).

The camera control unit 30 and the monitor 5 are mounted in a single housing as one imaging system 90.

### [Configuration of Camera Head Portion of Endoscope Camera]

A configuration of the camera head portion 11, which is the tip portion of the endoscope camera 10 will be described with reference to Fig. 4. Fig. 4 is a schematic diagram schematically illustrating a hardware configuration of the tip portion (camera head portion 11) of the endoscope illustrated in Fig. 2.

The camera head portion 11 of the endoscope camera 10 is formed in a cylindrical shape, and includes a proximal end portion connecting to a tip portion of the transmission cable 2. The camera head portion 11 mainly includes a holder 12 (lens barrel), a lens cover 13, a lens 15 (an example of an optical system), the image sensor 16 (an example of the image sensor), and the acceleration sensor 17 (an example of the posture control sensor).

The holder 12 is formed in a substantially cylindrical shape, is located at a tip of a direction in which the endoscope camera 10 is inserted, and is provided with the lens cover 13 exposed on a tip surface thereof. The lens 15 is formed in a disk shape and is used as a part of the optical system. The lens 15 is adjacent to the lens cover 13 and an optical axis of the lens 15 is arranged so as to substantially coincide with an axis of the holder 12. Each of the lens cover 13 and the lens 15 is arranged and held on a tip side of the holder 12, and captures imaging light (in other words, light from the observation site (subject) such as a blood vessel in the subject is incident). The lens 15 is held in a state where a periphery thereof is covered by the holder 12. A surface of the lens 15 may be fixed integrally with the lens cover 13.

The image sensor 16 and the acceleration sensor 17 are arranged on a proximal end side of the holder 12. The image sensor 16 and the acceleration sensor 17 are each held in a state of peripheries thereof being covered by the holder 12. The image sensor 16 and the acceleration sensor 17 include a plurality of terminals, and the plurality of terminals are each provided with an electric wire and are electrically connected to the transmission cable 2.

The image sensor 16 is arranged closer to a tip side of the camera head portion 11 than the acceleration sensor 17, and is arranged adjacent to the lens 15. That is, the image sensor 16 is arranged between the lens 15 and the acceleration sensor 17. The image sensor 16 is a solid-state imaging element such as a charged-coupled device (CCD) or a complementary metal-oxide-semiconductor (CMOS), and forms an image of light from the subject (for example, the affected area of the observation site) on an imaging surface through the lens 15, converts the formed optical image into an electric signal, and outputs the signal of the image. Since the lens 15 and the image sensor 16 function integrally, in the present embodiment, a configuration including the lens 15, which is the optical system, and the image sensor 16 is also referred to as an "imaging unit 14".

The acceleration sensor 17 is arranged closer to a proximal end side of the camera head portion 11 than the image sensor 16. The acceleration sensor 17 converts acceleration or impact including the gravitational acceleration (acceleration in a direction along a direction of gravity) acting on the camera head portion 11 into an electric signal, and outputs the signal of the acceleration acting on the camera head portion 11 and the like. In the present embodiment, as described above, the acceleration sensor coordinate system Σg is set for the acceleration sensor 17. Therefore, the acceleration sensor 17 is provided so that acceleration signals (gravitational acceleration and/or impact signals) in each of three directions (X, Y, and Z axes) orthogonal to each other, for example, a front-rear direction, an up-down direction, and a left-right direction of the tip portion (camera head portion 11), can be periodically detected for each of components thereof. The acceleration sensor 17 outputs a detection result for each of the X, Y, and Z components in the acceleration sensor coordinate system Σg. Since the acceleration sensor 17 can also detect the gravitational acceleration, a posture of the tip portion (the camera head portion 11 in the present embodiment) to which the acceleration sensor 17 is attached can be calculated by being divided into components in the three directions in the standard coordinate system Σs.

An inside of the camera head portion 11 (tip portion) is filled with a sealing material so that there is no gap. By this filling, the lens cover 13, the lens 15, the image sensor 16, and the acceleration sensor 17 are firmly fixed inside the camera head portion 11 of the endoscope camera 10. Therefore, the acceleration sensor 17 is arranged so that a relative position between the acceleration sensor 17 and the image sensor 16 is fixed. That is, relative positions of the image sensor 16 and the acceleration sensor 17 are fixed to each other. Therefore, when a coordinate system is set for the image sensor 16, the coordinate system can be regarded as a coordinate system obtained by simply translating the acceleration sensor coordinate system Σg. That is, based on the X, Y, and Z components in the acceleration sensor coordinate system Σg detected by the acceleration sensor 17, that is, posture information in the three directions of the camera head portion 11, it is possible to accurately calculate an amount of deviation (deviation angle, rotation position Pθ (described later)) in the up-down direction of the image.

### [Functional Configuration of Camera Control Unit]

A functional configuration of the camera control unit 30 will be described with reference to Fig. 5. Fig. 5 is a functional block diagram illustrating processing by the processor 31 of the camera control unit 30 illustrated in Fig. 4.

As shown in Fig. 5, the camera control unit 30 includes a multiplex signal demodulation circuit 40, a signal multiplex transmission circuit 41, a camera synchronization signal generation circuit 42, a pre-processing unit 43, a signal amplification processing unit 44, a YC separation processing and RGB conversion unit 45, a correction processing unit 46, a first gain level adjusting unit 47, a second gain level adjusting unit 48, an area detection processing unit 49, an area generation unit 50, and a fog feature amount detection data addition and averaging and storage unit 51. The camera control unit 30 further includes an encoder processing unit 52, an area display signal replacement mixing and selection unit 53, an indicator generation unit 54, a control unit 55, a data storage unit 56, an external switch 57, and an external communication unit 59. The external switch 57 includes a camera setting change button 58.

The multiplex signal demodulation circuit 40 demodulates and outputs multiplexed power supply signal and drive signal of the image sensor 16 and setting signals to each circuit output from the image sensor 16 of the camera head portion 11. The signal multiplex transmission circuit 41 multiplexes the signal of the image from the image sensor 16 and each information signal inside the camera head portion 11, and outputs the multiplexed signal.

The pre-processing unit 43 performs a pre-processing such as a noise removal processing and a color separation processing by a pixel filter arrangement on the signal of the image output from the image sensor 16 of the camera head portion 11. The signal amplification processing unit 44 performs a signal processing such as amplification on the signal of the image preprocessed by the pre-processing unit 43.

The first gain level adjusting unit 47 adjusts an amplification factor (that is, a gain) of the signal of the image according to a set value from the control unit 55.

The YC separation processing and RGB conversion unit 45 separates a luminance signal (Y signal) and a color signal (C signal) from the signal of the image from the signal amplification processing unit 44, and converts the color signal into an RGB signal. The YC separation processing and RGB conversion unit 45 outputs the separated Y signal to the area display signal replacement mixing and selection unit 53 via the correction processing unit 46.

The correction processing unit 46 generates color difference signals (R-Y), (G-Y), and (B-Y) using the RGB signal output from the YC separation processing and RGB conversion unit 45, and outputs the color difference signals to the encoder processing unit 52.

The second gain level adjusting unit 48 adjusts a gain of a circuit that can divide and set an input level for each area according to a set value read by the control unit 55 with respect to the correction processing unit 46. The set value read by the control unit 55 is stored and retained in the data storage unit 56.

The data storage unit 56 stores and retains a correction set value for intravascular use as the above-mentioned set value. The data storage unit 56 also stores and retains intravascular feature amount detection data.

The area generation unit 50 generates a display area frame signal based on area designation coordinates output from the control unit 55, and outputs the display area frame signal to the area display signal replacement mixing and selection unit 53 and the fog feature amount detection data addition and averaging and storage unit 51.

The display area frame signal is a signal for displaying a correction detection area frame on a display panel of the monitor 5. A size of the correction detection area frame can be set freely or automatically. For example, when a diameter of the endoscope camera 10 is small and an imaging range is narrow, the size of the correction detection area frame is set to a small (appropriate) value. In a case where the correction detection area frame is automatically set, when the diameter of the endoscope camera 10 is small, the imaging range thereof is narrowed, so that it may be automatically selectable according to the diameter.

When the display area frame signal is input from the area generation unit 50, the area display signal replacement mixing and selection unit 53 mixes the display area frame signal with the Y signal from the YC separation processing and RGB conversion unit 45 and outputs the mixed signal to the encoder processing unit 52. When area display switching designation coordinates are input from the control unit 55, the area display signal replacement mixing and selection unit 53 determines a position of the correction detection area frame according to information of the coordinates.

The area display switching designation coordinates are determined by an operation amount of the camera setting change button 58 in a menu for moving the correction detection area frame. The correction detection area frame is moved to a right direction by a right button among left, right, up, and down buttons of the camera setting change button 58 being pressed, and is moved to a left direction by a left button being pressed, for example. The correction detection area frame is moved upward by an up button being pressed, and is moved downward by a down button being pressed.

The area detection processing unit 49 detects an area where a feature amount is to be detected, for example, an area of a part of the image where fog is generated (that is, a display area of the correction detection area frame) from each signal from the YC separation processing and RGB conversion unit 45, and outputs a detection result to the fog feature amount detection data addition and averaging and storage unit 51.

The fog feature amount detection data addition and averaging and storage unit 51 adds fog feature amount detection data for the display area of the part of the image where fog is generated, which is detected by the area detection processing unit 49 based on the display area frame signal input from the area generation unit 50, calculates to acquire an average value and a statistical frequency (statistical data), and stores the above results.

The indicator generation unit 79 generates character data and graphic data to be superimposed and displayed on the image according to indicator display designation of characters and/or figures and the like input from the control unit 55. In the present embodiment, as an example of the character data, X, Y, and Z coordinate values indicating a position of the camera head portion 11 in the standard coordinate system Σs are generated (see Figs. 8A to 8C). As an example of the graphic data, the animation icon AI of the compass showing the posture information of the image in the standard coordinate system Σs is generated (see Figs. 8A to 8C). These generated indicators are output to the encoder processing unit 52 so as to be superimposed on the signal of the image via the area display signal replacement mixing and selection unit 53. The coordinate values of X, Y, and Z indicating the position of the camera head portion 11 in the standard coordinate system Σs, which is an example of the character data, are calculated by, for example, double integrating detected values of the acceleration sensor 17 of the camera head portion 11.

The camera synchronization signal generation circuit 42 generates an image sensor drive signal for driving the image sensor 16 of the camera head portion 11, and a synchronization signal and a vertical synchronization signal for signal processing used in operation of each unit.

The external communication unit 59 communicates with an external device (for example, a computer) and enables the external device to select a display area and read pixel information or color space information of the selected display area. Here, the display area selected by the external device is not limited to one, and a plurality of display areas may be simultaneously selected. Since the camera control unit 30 includes the external communication unit 59, it is possible to perform key operations on the external device side in addition to key operations performed by a single unit, and a degree of freedom of operations is improved. For example, it is possible to interactively select the display area and perform color correction using a mouse-type pointer or GUI, and change setting of the camera control unit 30 and store the setting.

The control unit 55 comprehensively controls operations of each unit of the camera control unit 30 in addition to the above-mentioned processing units.

For example, the control unit 55 reads out the statistical data and a luminance level stored in the fog feature amount detection data addition and averaging and storage unit 51, calculates a gain set value used in a fog correction processing, and sets the gain set value in the gain level adjusting units.

The fog correction processing is a known technique, and various processing are exemplified. For example, in the fog correction processing, in order to increase a contrast of an entire screen, which is reduced due to concentration of luminance histogram in a middle tone due to generation of fog, the set value of the correction processing (gain setting value in the present embodiment) is changed so as to flatten the luminance histogram. The external switch 57 including the camera setting change button 58 is connected to the control unit 55. The control unit 55 communicates with a computer, which is an external device, via the external communication unit 59, and enables the computer to control the camera control unit 30. The external switch 57 including the camera setting change button 58 is connected to the control unit 55.

In the present embodiment, as will be described later, the control unit 55 acquires an acceleration signal, that is, the posture information in the three directions, from the acceleration sensor 17 of the camera head portion 11 (endoscope camera 10) via the multiplex signal demodulation circuit 40. In an initial state that is before the endoscope is inserted into the subject, the control unit 55 stores the posture information in the three directions in the data storage unit 56 as calibration data.

Then, after the endoscope camera 10 is inserted into the subject, the control unit 55 calculates the amount of deviation (the deviation angle) between the acceleration sensor coordinate system Σg and the standard coordinate system Σs in the Z direction based on the posture information in the three directions, in other words, the amount of deviation in the up-down direction of the image captured by the imaging unit 14. Based on the calculation result, the control unit 55 assigns the indicator display designation to the indicator generation unit 54. By this assigning, the camera control unit 30 can display the image on the monitor 5 in association with the indicator indicating presence or absence of deviation in the up-down direction of the image.

### [Processing Procedure for Superimposing and Displaying Indicator on Image]

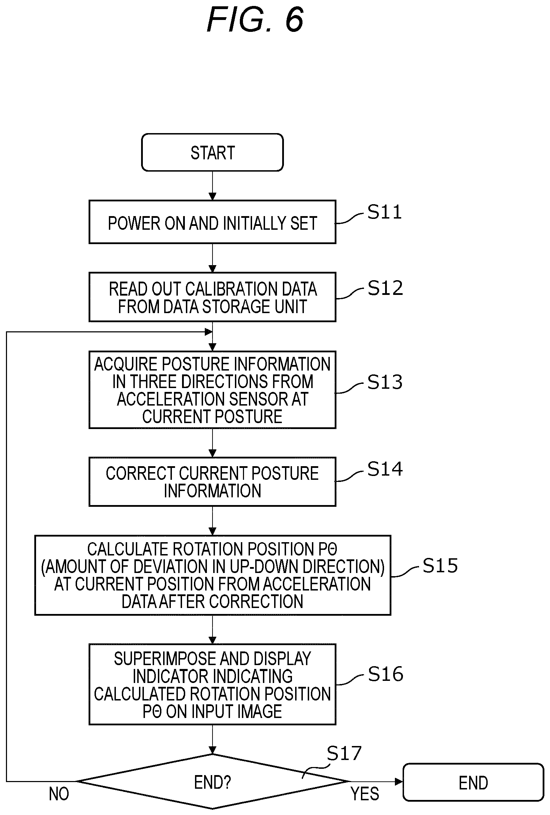

A processing procedure of the camera control unit 30 superimposing and displaying the indicator on the image will be described with reference to Fig. 6. Fig. 6 is a flow chart illustrating the processing procedure for superimposing and displaying the indicator on the image.

As shown in Fig. 6, when the endoscope system 1 is powered on, the endoscope camera 10, the pullback device, the relay device 4, the camera control unit 30, and the monitor 5 (see Fig. 3) are initially set. During this time, in the endoscope camera 10, at least the image sensor 16 and the acceleration sensor 17 are initially set (S11). After the initial setting, the control unit 55 of the camera control unit 30 reads out the above-mentioned calibration data from the data storage unit 56 (S12). This calibration data is the posture information in the three directions of the camera head portion 11 of the endoscope camera 10 in the initial state before the endoscope is inserted into the subject, and serves as a reference value for calculating the posture information in the three directions after the endoscope is inserted. The posture information in the three directions of the calibration data is coordinate-converted from the acceleration sensor coordinate system Σg to the standard coordinate system Σs.

After the endoscope is inserted into the subject, at a current insertion position, for example, at the first insertion position P0 or the second insertion position P1, the control unit 55 acquires an acceleration signal from the acceleration sensor 17 of the camera head portion 11 and detects current posture information of the camera head portion 11 in the three directions (S13). Since the posture information detected by the acceleration sensor 17 is a value in the acceleration sensor coordinate system Σg, the control unit 55 converts the posture information in the acceleration sensor coordinate system Σg into the posture information in the standard coordinate system Σs. The control unit 55 subtracts a value of the calibration data (initial posture information) from the converted posture information to correct the current posture information (S14).

The control unit 55 calculates the amount of deviation in the up-down direction of the image captured by the image sensor 16, that is, the rotation position Pθ, based on the corrected current posture information in the three-direction, and determines the presence or absence of the amount of deviation (S15). The control unit 55 designates indicator display such as characters and/or graphics to the indicator generation unit 54. By this indicator display designation, the coordinate values of X, Y, and Z indicating the position of the camera head portion 11 in the standard coordinate system Σs are generated as the character data. As the graphic data, an animation icon of a compass showing the posture of the image in the standard coordinate system Σs is generated. The control unit 55 superimposes and displays the indicator indicating the calculated rotation position Pθ on the image output from the image sensor 16 (S16).

The control unit 55 determines whether or not the processing flow should be ended (S17). If it is determined that the processing should not be ended (NO in S17), the processing flow returns to step S13, and the steps S13 to S16 described above are repeatedly executed.

That is, the steps S13 to S16 described above are sequentially executed as long as it is determined that the entire processing flow should not be ended, and the indicator is updated and displayed on the image during this period. Therefore, for the user such as a doctor of the endoscope system 1, it is possible to easily grasp how much the camera head portion 11 (tip portion) of the endoscope camera 10 is rotated around an axis thereof, in other words, how much the camera head portion 11 is deviated in the up-down direction. On the other hand, if it is determined that the processing flow should be ended (or terminated) (YES in S17), the entire processing flow is ended (END).

### [Use Case of Superimposing Indicator on Image]

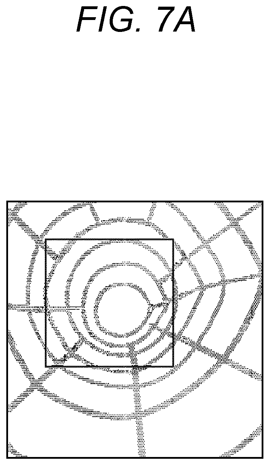

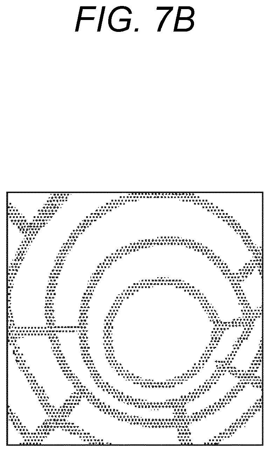

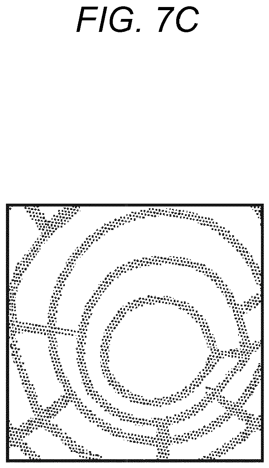

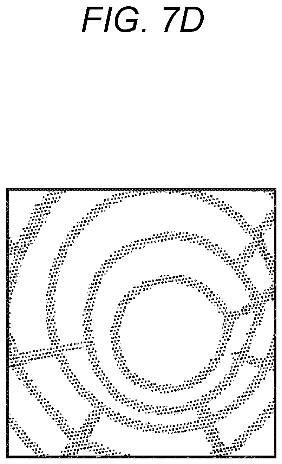

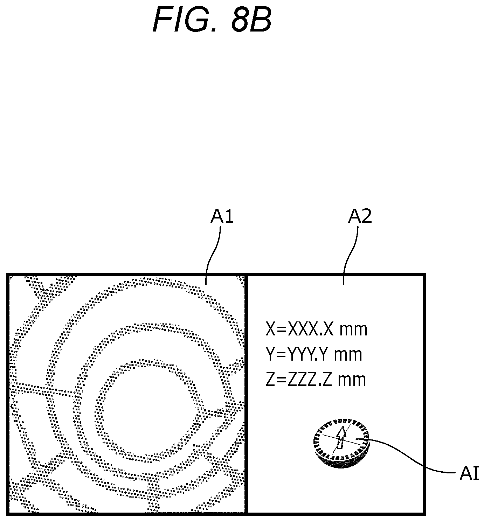

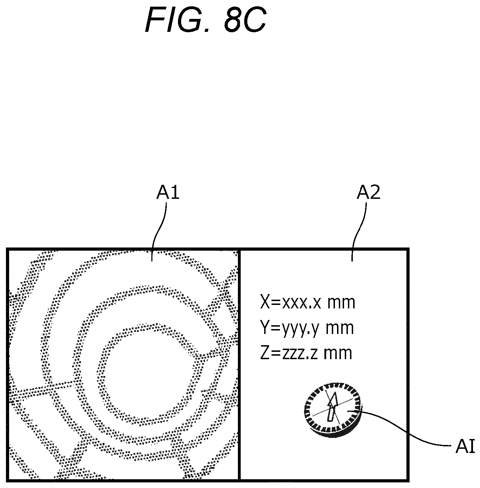

A use case of superimposing the indicator on the image will be described with reference to Figs. 7A, 7B, 7C, 7D, 8A, 8B, and 8C. Figs. 7A to 7D are schematic diagrams illustrating the image at each insertion position before the processing illustrated in Fig. 6 is executed. Figs. 8A to 8C are schematic diagrams illustrating the image after the processing illustrated in Fig. 6 is executed on the image illustrated in Figs. 7A to 7D.

Specifically, Fig. 7A is an example of a blood vessel image taken at the first insertion position P0 illustrated in Fig. 1. Fig. 7B is an example of a blood vessel image taken at the second insertion position P1 (see Fig. 1) further inserted from the first insertion position P0. Fig. 7C is an example of a blood vessel image taken by rotating 10 degrees clockwise of the drawing when the posture of Fig. 7B at the second insertion position P1 is used as a reference. Fig. 7D is an example of a blood vessel image taken by rotating 10 degrees counterclockwise of the drawing when the posture of Fig. 7B at the second insertion position P1 is used as a reference.

Fig. 8A is an example of a screen in which the processing illustrated in Fig. 6 is executed on the blood vessel image illustrated in Fig. 7B so that the indicator is superimposed and displayed. Fig. 8B is an example of a screen in which the processing illustrated in Fig. 6 is executed on the blood vessel image illustrated in Fig. 7C so that the indicator is superimposed and displayed. Fig. 8C is an example of a screen in which the processing illustrated in Fig. 6 is executed on the blood vessel image illustrated in Fig. 7D so that the indicator is superimposed and displayed.

As shown in Fig. 7A, the endoscope camera 10 is inserted into the subject, and a state of the observation site in the subject at the first insertion position P0 (see Fig. 1) is imaged. At the first insertion position P0, the user such as a doctor can intuitively understand how much the endoscope camera 10 is rotated about the axis immediately after being inserted in the subject. Therefore, when the blood vessel image of Fig. 7A is displayed on the monitor 5, the user can smoothly operate the endoscope camera 10 without being aware of deviation in the up-down direction and the vertical direction (gravity direction) of the blood vessel image.

When the endoscope camera 10 is further inserted into a deep side of the subject and the endoscope camera 10 is positioned at the second insertion position P1 (see Fig. 1), as shown in Figs. 7B to 7D, it is difficult to extract special marks or feature points from the blood vessel image. Therefore, it is difficult for the user to understand how much the endoscope camera 10 located on the deep side is rotated about the axis in the same situation based on the images illustrated in Figs. 7B to 7D.

Therefore, in the present embodiment, the camera control unit 30 calculates the presence or absence of the deviation in the up-down direction of the image captured by the imaging unit 14 based on the posture information in the three directions detected by the acceleration sensor 17 arranged on the camera head portion 11 of the endoscope camera 10.

As shown in Figs. 8A to 8C, the camera head portion 11 displays the indicator indicating the presence or absence of deviation in the up-down direction of the blood vessel image in association with the blood vessel image on the monitor 5 according to the calculation result. Specifically, on the screen of the monitor 5, an indicator display area A2 (on a right side in Figs. 8A to 8C) is arranged adjacent to an image area A1 (on a left side in Figs. 8A to 8C) on which the blood vessel image is displayed. As a character indicator, coordinate values of X, Y, and Z indicating the position of the camera head portion 11 in the standard coordinate system Σs are displayed on one side of the indicator display area A2 (an upper right side in Figs. 8A to 8C). As a graphic indicator, an animation icon of a compass showing the posture of the blood vessel image in the standard coordinate system Σs is displayed (on a lower right side in Figs. 8A to 8C).

In this way, by displaying the image on the monitor 5 in association with the indicator indicating the presence or absence of deviation in the up-down direction of the image, the user such as a doctor can easily understand how much the endoscope camera 10 is deviated around the axis in the up-down direction with the endoscope camera 10 inserted in the subject, regardless of whether the insertion position of the endoscope camera 10 is on the deep side or a front side, and it is possible to improve operability of the endoscope system 1. That is, it is possible to efficiently detect the presence or absence of deviation in the up-down direction of the tip portion of the endoscope and support implementation of appropriate medical practice. Therefore, in the medical field, it is possible to reduce occurrence of operation errors by the user such as a doctor and improve medical safety for patients.

As described above, the endoscope system 1 of the first embodiment includes: the endoscope camera 10 (an example of the endoscope) that can be inserted in and pulled out from the subject (a blood vessel of a human body and the like), and includes the tip portion provided with the imaging unit 14 including the lens 15 (an example of the optical system) and the image sensor 16 (an example of the image sensor); the acceleration sensor 17 (an example of the posture control sensor) that is arranged at the tip portion, and detects the posture information in at least three directions, the front-rear direction, the up-down direction, and the left-right direction, of the tip portion; and the camera control unit 30 (an example of the calculation device) that calculates the presence or absence of deviation in the up-down direction of the image captured by the imaging unit 14 based on the posture information in the three directions detected by the acceleration sensor 17. The camera control unit 30 displays the image on the monitor 5 in association with the indicator indicating the presence or absence of deviation in the up-down direction of the image.

In this way, by displaying the image on the monitor 5 in association with the indicator indicating the presence or absence of deviation in the up-down direction of the image, the user such as a doctor can easily understand how much the endoscope camera 10 is deviated around the axis in the up-down direction with the endoscope camera 10 inserted in the subject, regardless of whether the insertion position of the endoscope camera 10 is on the deep side or a front side, and it is possible to improve operability of the endoscope system 1. That is, it is possible to efficiently detect the presence or absence of deviation in the up-down direction of the tip portion of the endoscope and support the implementation of appropriate medical practice. Therefore, in the medical field, it is possible to reduce the occurrence of operation errors by the user such as a doctor and improve the medical safety for patients.

According to the endoscope system 1 of the first embodiment, the acceleration sensor 17 (an example of the posture control sensor) is arranged so as to have a fixed relative position with respect to the image sensor 16 (an example of the image sensor). Therefore, when a coordinate system is set for the image sensor 16, the coordinate system can be regarded as a coordinate system obtained by simply translating the acceleration sensor coordinate system Σg. That is, based on the X, Y, and Z components in the acceleration sensor coordinate system Σg including the gravitational acceleration detected by the acceleration sensor 17, that is, the posture information in the three directions of the camera head portion 11, it is possible to accurately calculate the amount of deviation (deviation angle, rotation position Pθ) in the up-down direction of the image.

According to the endoscope system 1 of the first embodiment, the imaging unit 14 and the acceleration sensor 17 (an example of the posture control sensor) are fixed by the sealing material filled in the tip portion. Therefore, since the image sensor 16 and the acceleration sensor 17 are firmly fixed inside the camera head portion 11 of the endoscope camera 10, the amount of deviation of the image in the up-down direction can be calculated more accurately based on the posture information of the camera head portion **11** in the three directions.

According to the endoscope system 1 of the first embodiment, the camera control unit 30 (an example of the calculation device) stores in the memory 32, as the calibration data, the posture information in the three directions detected by the acceleration sensor 17 (an example of the posture control sensor) in the initial state that is before the endoscope camera 10 (an example of the endoscope) is inserted into the subject, and corrects, using the calibration data, the posture information in the three directions detected by the acceleration sensor 17 after the endoscope camera 10 is inserted into the subject. Therefore, the amount of deviation of the image in the up-down direction can be calculated more accurately based on the posture information of the camera head portion 11 in the three directions, and the presence or absence of deviation in the up-down direction of the tip portion of the endoscope can be detected more accurately.

### (Second Embodiment)

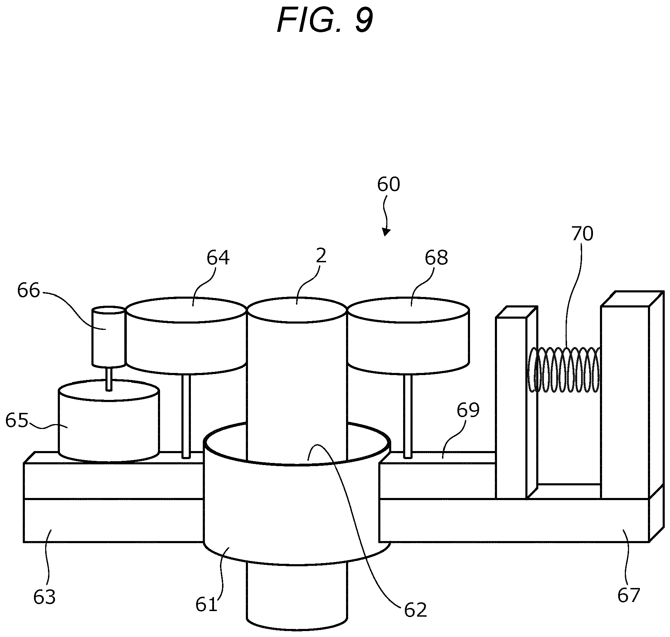

A second embodiment according to the present disclosure will be described with reference to Figs. 9 to 12. Since descriptions of the same or equivalent parts as those in the above-described first embodiment are duplicated, the same reference numerals may be given to the drawings to omit or simplify the descriptions.

### [Configuration of Rotary Drive Device 60]

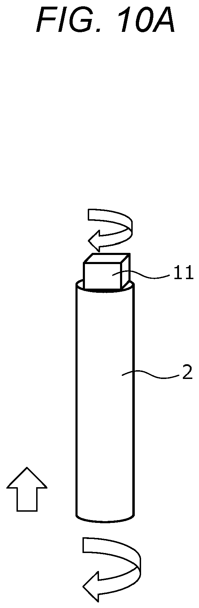

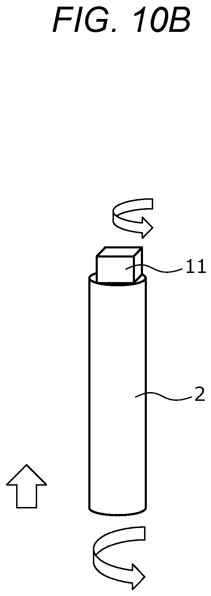

A rotary drive device 60 (an example of a drive device) that applies a rotational force around an axis of the transmission cable 2 to the tip portion will be described with reference to Figs. 9, 10A, and 10B. Fig. 9 is a schematic diagram illustrating a hardware configuration of a rotary drive device 60 according to the second embodiment. Fig. 10A is a schematic diagram illustrating a first example of a case where the transmission cable 2 is rotated by the rotary drive device 60. Fig. 10B is a schematic diagram illustrating a second example of the case where the transmission cable 2 is rotated by the rotary drive device 60.

The endoscope system 1 of the present embodiment further includes the rotary drive device 60 as compared with the above-described first embodiment.

The rotary drive device 60 is attached to the transmission cable 2 by being arranged near the camera control unit 30 or the pullback device on the proximal end portion of the transmission cable 2 (a side opposite to the tip portion to which the endoscope camera 10 is attached). The rotary drive device 60 fixes the proximal end portion of the transmission cable 2 and applies a rotational force around the axis (in an axial direction) of the transmission cable 2 to the tip portion via the transmission cable 2. As a result, it is possible to change and adjust the posture (that is, the rotation angle) around the axis of the camera head portion 11 attached to the tip portion of the transmission cable 2 (see below).

As shown in Fig. 9, the rotary drive device 60 includes a cable holding portion 61 that rotatably holds the transmission cable 2 around the axis, a movable shaft holding portion 63 provided in connection with the cable holding portion 61, and a passive shaft holding portion 67 also provided in connection with the cable holding portion 61. The cable holding portion 61 is formed with an insertion hole 62 (not shown) through which the transmission cable 2 is inserted. The movable shaft holding portion 63 and the passive shaft holding portion 67 are arranged so as to face each other with an axial center of the insertion hole 62 of the cable holding portion 61 interposed therebetween.

When the cable holding portion 61 holds the transmission cable 2, the transmission cable 2 is inserted through the insertion hole 62, and the transmission cable 2 is rotatably held in the insertion hole 62 so that the axis thereof substantially coincides with the axial center of the insertion hole 62.

The movable shaft holding portion 63 is provided with a movable roller 64, and the movable roller 64 is axially supported so that an axial center thereof is substantially parallel to the insertion hole 62. The movable roller 64 includes an outer peripheral portion attached with a urethane sponge, and the movable roller 64 is in contact with the transmission cable 2 on the outer peripheral surface thereof. By attaching the urethane sponge, sliding (exceeding a frictional force) between the movable roller 64 and the transmission cable 2 is allowed so as to prevent unreasonable or excessive rotational force (see below) from being transmitted to the transmission cable 2, and it is possible to, for example, avoid damage to the observation site in the subject.

The movable shaft holding portion 63 is provided with a motor 65, and the motor 65 includes an output shaft, whose tip is provided with an output roller 66. An outer peripheral surface of the output roller 66 is in contact with an outer peripheral surface of the movable roller 64, and a driving force of the motor 65 is transmitted to the movable roller 64. As a result, the driving force of the motor 65 is transmitted to the proximal end portion of the transmission cable 2 as the rotational force around the axis of the transmission cable 2 via the output roller 66 and the movable roller 64.

The passive shaft holding portion 67 is provided with a passive roller 68, and same as the movable roller 64, the passive roller 68 is also rotatably and axially supported so that an axial center thereof is substantially parallel to the insertion hole 62. The passive roller 68 also includes an outer peripheral portion attached with a urethane sponge, and the passive roller 68 is in contact with the transmission cable 2 on the outer peripheral surface thereof.

The passive shaft holding portion 67 further includes a slider portion 69. The slider portion 69 directly holds the passive roller 68 and moves the passive roller 68 so as to be accessible while keeping the axial center of the passive roller 68 parallel to the axis of the transmission cable 2. An urging portion 70 made of an elastic member such as a compression spring is attached to the slider portion 69. The slider portion 69 is urged and moved so that the passive roller 68 is always close to an axial center of the transmission cable 2. By this urging movement, a predetermined frictional force can be adjusted so as to be generated on a contact surface among the transmission cable 2, the movable roller 64, and the passive roller 68. Therefore, the movable roller 64 and the passive roller 68 are provided so as to be able to be sandwiched so that no unreasonable or excessive rotational force is transmitted to the proximal end portion of the transmission cable 2 held by the cable holding portion 61.

As shown in Fig. 10A, when a rotational force is applied around the axis in a forward direction (for example, clockwise) to the proximal end portion of the transmission cable 2 by being driven by the motor 65 of the rotary drive device 60, the camera head portion 11 also rotates clockwise around the axis with a delay corresponding to a distance between the proximal end portion and the tip portion (camera head portion 11).

In contrast, as shown in Fig. 10B, when a rotational force is applied to the proximal end portion of the transmission cable 2 around the axis in a reverse direction (for example, counterclockwise) different from that in Fig. 10A by being driven by the motor 65 of the rotary drive device 60, similarly, the camera head portion 11 rotates around the axis counterclockwise with the delay corresponding to the distance between the proximal end portion and the tip portion. By applying such rotation, the camera head portion 11 of the endoscope camera 10 is similarly rotated, and as a result, the image captured by the image sensor 16 is also rotated so that it is possible to image the observation site in the subject at a desired rotation angle set in advance.

In order to control the rotary drive device 60 configured in this way, the camera control unit 30 acquires (calculates) the rotational force that rotates the tip portion around the axis (in the axial direction) based on a difference between the posture information in the three directions detected by the acceleration sensor 17 and a predetermined posture state. Then, the camera control unit 30 instructs the rotary drive device 60 to rotate the tip portion around the axis based on a calculated result of the rotational force. That is, as described below, the camera control unit 30 calculates a control value for the motor 65 of the rotary drive device 60 (hereinafter also referred to as "motor 65 control value"), and controls the rotational force of the motor 65 based on the motor 65 control value, thereby defining the rotational position of the camera head portion 11 of the endoscope camera 10 at a desired rotational angle. In the data storage unit 56 of the camera control unit 30, a value of a target angle Nθ (desired rotation angle in the standard coordinate system Σs) of the camera head portion 11 of the endoscope camera 10 with respect to the up-down direction is set (stored and retained) in advance.

### [Processing Procedure to Control Rotation of Transmission Cable around Axis]

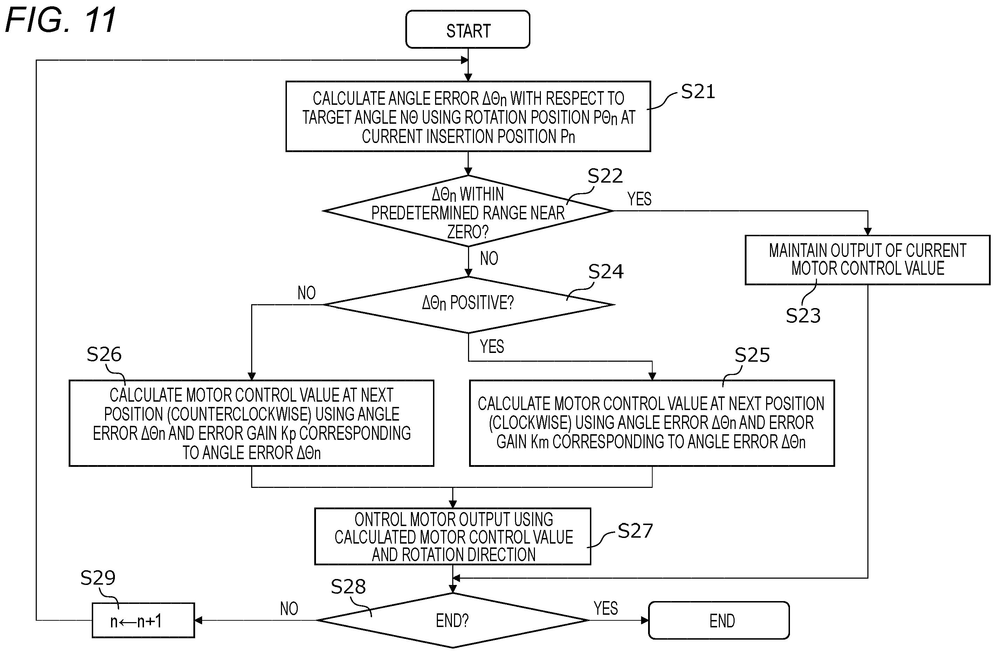

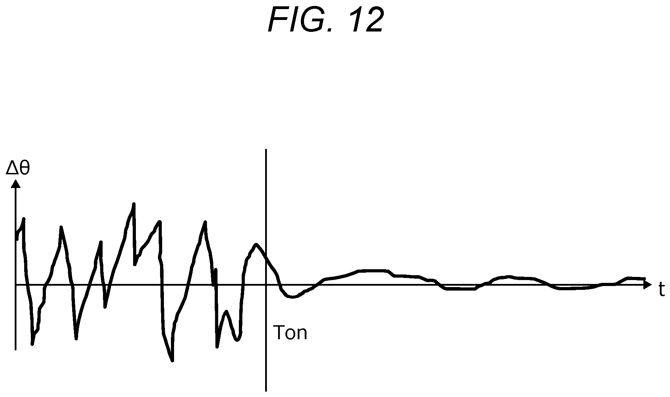

A processing procedure for controlling the rotation of the transmission cable 2 around the axis, which is executed by the camera control unit 30 of the present embodiment, will be described with reference to Figs. 11 and 12. Fig. 11 is a flow chart illustrating the processing procedure for controlling the rotation of the transmission cable 2 around the axis. Fig. 12 is a graph illustrating a case where the rotational force of the transmission cable 2 around the axis is controlled and converged to a predetermined value.

A processing flow shown in Fig. 11 is sequentially executed at each of the first insertion positions P0, ..., Pn (wherein n is 0 or a natural number), ... in an insertion path when the endoscope camera 10 is inserted in or pulled out from the observation site in the subject. That is, a symbol such as "Pθn" and "Δθn" means a value corresponding to the insertion position Pn (a value when the insertion position is at Pn). "Pn + 1" means a next insertion position after a predetermined period is lapsed.

As shown in Fig. 11, the camera control unit 30 calculates the rotation position Pθn at the current insertion position Pn based on the posture information in the three directions from the acceleration sensor 17 (see step S15 in Fig. 6 for calculation of the rotation position Pθ). The camera control unit 30 reads out the target angle Nθ from the data storage unit 56, and calculates an angle error Δθn (= Pθn - Nθ), which is a difference between the current rotation position Pθn and the target angle Nθ (S21).

Based on a calculation result of the angle error Δθn, the camera control unit 30 determines whether or not the angle error Δθn is within a predetermined range near 0 (zero) (S22). According to a determination result, when it is determined that the angle error Δθn is within a predetermined range near 0 (zero) (YES in S22), the camera control unit 30 determines that no control (change or update) instruction is given to the motor 65 of the rotary drive device 60, and maintains output of the current motor 65 control value (S23). Then, the processing flow proceeds to step S28. By determining whether or not such an angle error Δθn is within a predetermined range near 0 (zero), in other words, by setting a dead zone, a slight rotational force is prevented from being applied to the tip portion (camera head portion 11) of the endoscope camera 10. As a result, it is possible to reduce minute movements in the camera head portion 11 and prevent screen sickness of the user such as a doctor or deterioration of the endoscope camera 10 over time.

When it is determined that the angle error Δθn is not within a predetermined range near 0 (zero) (NO in S22), the camera control unit 30 determines whether or not the angle error Δθn is positive (+) (S24). That is, the camera control unit 30 acquires (calculates) the direction of rotation around the axis of the camera head portion 11 (tip portion) based on the above-mentioned difference (angle error Δθn). According to a calculation result, when the angle error Δθn is determined to be positive (+) (YES in S24), the camera control unit 30 uses the angle error Δθn and an error gain Km (coefficient) corresponding to the angle error Δθn to calculate a control value for the motor 65 of the rotary drive device 60 in the forward direction, for example, clockwise (see Fig. 10A), at the next insertion position (S25). For example, the motor 65 control value in this case is appropriately defined as a value linearly related to a value obtained by multiplying the angle error Δθn and the error gain Km by a servo control system such as PD control or PID control. Then, as shown in Fig. 12, by such servo control, the above-mentioned angle error Δθn at the current insertion position Pn is converged to a range of a steady error with respect to 0 "zero".

The explanation will be continued by returning to Fig. 11. When it is determined that the angle error Δθn is not positive (+), that is, is negative (-) (NO in S24), in this case, the camera control unit 30 calculates a control value for the motor 65 of the rotary drive device 60 in a reverse direction with respect to that in step S25, for example, counterclockwise (see Fig. 10B), at the next insertion position (S25) using the angle error Δθn and an error gain Kp (coefficient) corresponding to the angle error Δθn, Similarly, the motor 65 control value in this case is also defined as a value linearly related to a value obtained by multiplying the angle error Δθn and the error gain Kp.

In this way, when the motor 65 control value in the forward direction or the reverse direction is calculated (S25, S26), the control unit applies a rotational force to the proximal end portion of the transmission cable 2 around the axis clockwise or counterclockwise by driving and controlling the output of the motor 65 of the rotary drive device 60 (S27). By applying this rotation, the camera head portion 11 (tip portion) of the endoscope camera 10 also rotates in the same manner. Therefore, the posture control sensor can also rotate the image captured by the image sensor 16 whose relative position is fixed with respect to the image sensor, so as to capture an image of the observation site in the subject at a desired angle (that is, the target angle Nθ) in the preset standard coordinate system Σs, for example, in the normal up-down direction (for example, the image as illustrated in Fig. 8A can be obtained).

The camera control unit 30 determines whether or not the processing flow should be ended (S28). When it is determined that the processing flow should be ended (or is ended) (YES in S28), the entire processing flow ends (END). In contrast, when it is determined that the processing flow should not be ended (NO in S28), the processing flow updates the variable n to n + 1 and returns to step S21. That is, the insertion position of the endoscope camera 10 is updated to the next insertion position, and steps S21 to S27 are sequentially executed for each of the insertion positions. That is, the camera control unit 30 repeatedly executes the calculation of the rotational force and the instruction to the rotary drive device 60 (S21 to S27).

By such an execution, the camera control unit 30 repeatedly executes the above-mentioned calculation of the rotational force and the instruction to the rotary drive device 60, so that with the endoscope camera 10 inserted in the subject, the tip portion (camera head portion 11) can be sequentially rotated around the axis at a desired rotation angle with respect to the up-down direction. Therefore, the image captured by the image sensor 16 can also be rotated to image the observation site in the subject at a desired angle. Therefore, since the endoscope system 1 captures the observation site in a fixed posture with respect to the up-down direction, the user such as a doctor can appropriately and easily understand in which direction the observation site in the subject is imaged.

As described above, the endoscope system 1 of the second embodiment further includes the rotary drive device 60 (an example of the drive device) that fixes the transmission cable 2 connecting from the proximal end portion to the tip portion of the endoscope camera 10 (an example of the endoscope) by the proximal end portion side, and applies the rotational force around the axis (in the axial direction) of the transmission cable 2 to the tip portion via the transmission cable 2. The camera control unit 30 (an example of the calculation device) calculates the rotational force for rotating the tip portion around the axis (in the axial direction) based on the difference between the posture information in the three directions detected by the acceleration sensor 17 (an example of the posture control sensor) and a predetermined posture state, and instructs the rotary drive device 60 to rotate the tip portion around the axis (in the axial direction) based on a calculation result of the rotational force.

In this way, the control unit applies a rotational force to the proximal end portion of the transmission cable 2 around the axis clockwise or counterclockwise by driving and controlling the output of the motor 65 of the rotary drive device 60. By applying such rotation, the camera head portion 11 (tip portion) of the endoscope camera 10 is similarly rotated, and as a result, the image captured by the image sensor 16 is also rotated so that it is possible to image the observation site in the subject at a desired rotation angle set in advance. Therefore, since the endoscope system 1 captures the observation site in a fixed posture with respect to the up-down direction, the user such as a doctor can appropriately and easily understand in which direction the observation site in the subject is imaged. Therefore, it is possible to reduce the occurrence of operation errors by the user such as a doctor by supporting the implementation of appropriate medical practice, and improve the medical safety for patients.

According to the endoscope system 1 of the second embodiment, the camera control unit 30 (an example of the calculation device) further calculates the direction of rotation of the tip portion in the axial direction based on the difference (angle error Δθn). Therefore, the control unit can appropriately rotate the camera head portion 11 (tip portion) of the endoscope camera 10 around the axis in the forward direction (for example, clockwise) or the reverse direction (for example, counterclockwise) depending on a situation by driving and controlling the output of the motor 65 of the rotary drive device 60.

According to the endoscope system 1 of the second embodiment, the camera control unit 30 (an example of the calculation device) repeatedly executes the calculation of the rotational force and the instruction to the rotary drive device 60 (an example of the drive device). Therefore, the camera control unit 30 repeatedly executes the above-mentioned calculation of the rotational force and the instruction to the rotary drive device 60, so that with the endoscope camera 10 inserted in the subject, the tip portion (camera head portion 11) can be sequentially rotated around the axis at a desired rotation angle. As a result, convenience for the user such as a doctor is enhanced, and it is possible to support the implementation of more appropriate medical practice.

According to the endoscope system 1 of the second embodiment, the difference is a value in a predetermined range centered on zero. Therefore, in other words, by setting a dead zone, a slight rotational force is prevented from being applied to the tip portion (camera head portion 11) of the endoscope camera 10. As a result, it is possible to reduce minute movements in the camera head portion 11 and prevent the screen sickness of the user such as a doctor or deterioration of the endoscope camera 10 over time.

Other configurations and functions and effects are the same as those in the above-described first embodiment.

Although a plurality of embodiments have been described above with reference to the drawings, it is needless to say that the present disclosure is not limited to such examples. It will be apparent to those skilled in the art that various alterations, modifications, substitutions, additions, deletions, and equivalents can be conceived within the scope of the claims, and it should be understood that such changes also belong to the technical scope of the present disclosure. Further, components in the above-described embodiments may be optionally combined within a range not departing from the disclosure.

In the second embodiment, the rotary drive device 60 including the motor 65 has been described as the drive device for applying the rotational force around the axis of the transmission cable 2 to the tip portion (see Figs. 10A and 10B), but the present disclosure is not limited thereto. Instead of correcting the deviation of the tip portion of the endoscope camera 10 by rotating the cable on the proximal end side using such a motor 65, the deviation of the tip portion of the endoscope camera 10 may be corrected by arranging a plurality of artificial muscles (for example, a conductive polymer actuator) at the tip portion of the endoscope camera 10 and applying a voltage under control from the proximal end side.

### INDUSTRIAL APPLICABILITY

The present disclosure is useful as an endoscope system that efficiently detects presence or absence of deviation in an up-down direction of a tip portion of an endoscope and supports implementation of appropriate medical practice.

## Claims

1. An endoscope system (1) comprising:
an endoscope (10) that is configured to be inserted in and pulled out from a subject, and includes a tip portion (11) provided with an imaging unit (14) including an optical system (15) and an image sensor (16);
a posture control sensor (17) that is arranged at the tip portion (11) of the endoscope (10), and detects posture information in at least three directions, a front-rear direction, an up-down direction, and a left-right direction, of the tip portion (11); and
a calculation device (30) that calculates presence or absence of deviation in the up-down direction of an image captured by the imaging unit (14), based on the posture information in the three directions detected by the posture control sensor (17), wherein
the calculation device (30) displays an indicator indicating the presence or absence of deviation in the up-down direction of the image in association with the image on a monitor (5),
**characterized in that**
the calculation device (30) stores in a memory (32), as calibration data, the posture information in the three directions detected by the posture control sensor (17) in an initial state that is before the endoscope (10) is inserted into the subject, and corrects, using the calibration data, the posture information in the three directions detected by the posture control sensor (17) after the endoscope (10) is inserted into the subject,
wherein the calculation device (30) calculates an amount of deviation in the up-down direction based on the corrected posture information and displays, as the indicator, the posture of the tip portion (11) when the tip portion (11) during capturing an image is rotated from the initial state in the up-down direction.

2. The endoscope system (1) according to claim 1, wherein
the posture control sensor (17) is arranged so that a relative position of the posture control sensor (17) with respect to the image sensor (16) is fixed.

3. The endoscope system (1) according to claim 1, wherein
the imaging unit (14) and the posture control sensor (17) are fixed by a sealing material filled in the tip portion (11).

4. The endoscope system (1) according to claim 1, further comprising:
a drive device (60) that fixes a transmission cable (2) connecting from a proximal end portion to the tip portion (11) of the endoscope (10) at a proximal end portion side, and applies a rotational force in an axial direction of the transmission cable (2) to the tip portion (11) via the transmission cable (2), wherein
the calculation device (30) calculates the rotational force for rotating the tip portion (11) in the axial direction based on a difference between the posture information in the three directions detected by the posture control sensor (17) and a predetermined posture state, and instructs the drive device (60) to rotate the tip portion (11) in the axial direction based on a calculation result of the rotational force.

5. The endoscope system (1) according to claim 4, wherein
the calculation device (30) further calculates a direction in which the tip portion (11) is rotated in the axial direction based on the difference.

6. The endoscope system (1) according to claim 4, wherein
the calculation device (30) repeatedly executes the calculation of the rotational force and the instruction to the drive device (60).

7. The endoscope system (1) according to claim 4, wherein
the difference is a value in a predetermined range centered on zero.

## Patentansprüche

1. Endoskop-System (1) mit:
einem Endoskop (10), das so ausgestaltet ist, dass es in einen Patienten eingeführt und aus diesem herausgezogen werden kann, und das einen Spitzenabschnitt (11) umfasst, der mit einer Bildaufnahmeeinheit (14) versehen ist, die ein optisches System (15) und einen Bildsensor (16) umfasst,
einem Haltungssteuerungssensor (17), der am Spitzenabschnitt (11) des Endoskops (10) angeordnet ist und Haltungsinformationen in mindestens drei Richtungen, einer Vorwärts-Rückwärts-Richtung, einer Aufwärts-Abwärts-Richtung und einer Links-Rechts-Richtung, des Spitzenabschnitts (11) erfasst, und
einer Berechnungsvorrichtung (30), die ein Vorhandensein oder Nichtvorhandensein einer Abweichung in der Aufwärts-Abwärts-Richtung eines von der Bildaufnahmeeinheit (14) aufgenommenen Bildes basierend auf der vom Haltungssteuerungssensor (17) erfassten Haltungsinformationen in den drei Richtungen berechnet, wobei
die Berechnungsvorrichtung (30) einen Indikator anzeigt, der das Vorhandensein oder Fehlen einer Abweichung in der Aufwärts-Abwärts-Richtung des Bildes in Verbindung mit dem Bild auf einem Monitor (5) anzeigt,
**dadurch gekennzeichnet, dass**
die Berechnungsvorrichtung (30) in einem Speicher (32) als Kalibrierungsdaten die vom Haltungssteuerungssensor (17) erfassten Haltungsinformationen in den drei Richtungen in einem Anfangszustand, der vor dem Einführen des Endoskops (10) in den Patienten liegt, und unter Verwendung der Kalibrierungsdaten die vom Haltungssteuerungssensor (17) erfassten Haltungsinformationen in den drei Richtungen nach dem Einführen des Endoskops (10) in den Patienten korrigiert,
wobei die Berechnungsvorrichtung (30) einen Abweichungsbetrag in Aufwärts-Abwärts-Richtung anhand der korrigierten Haltungsinformationen berechnet und als den Indikator die Haltung des Spitzenabschnitts (11) anzeigt, wenn der Spitzenabschnitt (11) während der Aufnahme eines Bildes aus dem Anfangszustand in Aufwärts-Abwärts-Richtung gedreht ist.

2. Endoskop-System (1) nach Anspruch 1, wobei
der Haltungssteuerungssensor (17) so angeordnet ist, dass eine relative Position des Haltungssteuerungssensors (17) in Bezug auf den Bildsensor (16) fixiert ist.

3. Endoskop-System (1) nach Anspruch 1, wobei
die Bildaufnahmeeinheit (14) und der Haltungssteuerungssensor (17) durch ein in den Spitzenabschnitt (11) eingefülltes Dichtungsmaterial fixiert sind.

4. Endoskop-System (1) nach Anspruch 1, ferner mit:
einer Antriebsvorrichtung (60), die ein Übertragungskabel (2), das von einem proximalen Endabschnitt zum Spitzenabschnitt (11) des Endoskops (10) führt, an einer Seite eines proximalen Endabschnitt fixiert und über das Übertragungskabel (2) eine Drehkraft in axialer Richtung des Übertragungskabels (2) auf den Spitzenabschnitt (11) ausübt, wobei
die Berechnungsvorrichtung (30) die Drehkraft zum Drehen des Spitzenabschnitts (11) in axialer Richtung basierend auf einer Differenz zwischen den vom Haltungssteuerungssensor (17) erfassten Haltungsinformationen in den drei Richtungen und einem vorbestimmten Haltungszustand berechnet und die Antriebsvorrichtung (60) anweist, den Spitzenabschnitt (11) basierend auf einem Berechnungsergebnisses der Drehkraft in axialer Richtung zu drehen.

5. Endoskop-System (1) nach Anspruch 4, wobei
die Berechnungsvorrichtung (30) ferner basierend auf der Differenz eine Richtung berechnet, in der der Spitzenabschnitt (11) in axialer Richtung gedreht wird.

6. Endoskop-System (1) nach Anspruch 4, wobei
die Berechnungsvorrichtung (30) die Berechnung der Drehkraft und die Anweisung an die Antriebsvorrichtung (60) wiederholt ausführt.

7. Endoskop-System (1) nach Anspruch 4, wobei
die Differenz ein Wert in einem vorbestimmten Bereich um Null herum ist.

## Revendications

1. Système d'endoscope (1) comprenant:
un endoscope (10) qui est configuré pour être inséré dans et retiré d'un sujet, et comprend une portion de pointe (11) munie d'une unité de capture d'image (14) comprenant un système optique (15) et un capteur d'image (16);
un capteur de commande de posture (17) qui est disposé sur la portion de pointe (11) de l'endoscope (10), et détecte des informations de posture dans au moins trois directions, une direction avant-arrière, une direction haut-bas et une direction gauche-droite, de la portion de pointe (11) ; et
un dispositif de calcul (30) qui calcule une présence ou une absence d'une déviation dans la direction haut-bas d'une image capturée par l'unité de capture d'image (14), à partir des informations de posture dans les trois directions détectées par le capteur de commande de posture (17),
le dispositif de calcul (30) affiche un indicateur indiquant la présence ou l'absence de déviation dans la direction haut-bas de l'image, en association avec l'image affichée sur un moniteur (5),
**caractérisé en ce que**
le dispositif de calcul (30) stocke dans une mémoire (32), comme données de calibrage, les informations de posture dans les trois directions détectées par le capteur de commande de posture (17) dans un état initial, c'est-à-dire avant que l'endoscope (10) soit inséré dans le sujet, et corrige, à l'aide des données de calibrage, les informations de posture dans les trois directions détectées par le capteur de commande de posture (17) après l'insertion de l'endoscope (10) dans le sujet,
dans lequel le dispositif de calcul (30) calcule une amplitude de déviation dans la direction haut-bas à partir des informations de posture corrigées et affiche, comme indicateur, la posture de la portion de pointe (11) lorsque la portion de pointe (11) subit une rotation depuis l'état initial dans la direction haut-bas lors de la capture d'une image.

2. Système d'endoscope (1) selon la revendication 1, dans lequel
le capteur de commande de posture (17) est agencé de manière à ce qu'une position relative du capteur de commande de posture (17) par rapport au capteur d'image (16) soit fixe.

3. Système d'endoscope (1) selon la revendication 1, dans lequel
l'unité de capture d'image (14) et le capteur de commande de posture (17) sont fixés par un matériau d'étanchéité appliqué dans la portion de pointe (11).

4. Système d'endoscope (1) selon la revendication 1, comprenant en outre:
un dispositif d'entraînement (60) qui fixe un câble de transmission (2) menant d'une portion d'extrémité proximale à la portion de pointe (11) de l'endoscope (10) sur un côté de portion d'extrémité proximale, et applique une force de rotation dans une direction axiale du câble de transmission (2) sur la portion de pointe (11) par l'intermédiaire du câble de transmission (2), dans lequel
ledit dispositif de calcul (30) calcule la force de rotation nécessaire à la rotation de la portion de pointe (11) dans la direction axiale, à partir d'une différence entre les informations de posture dans les trois directions détectées par le capteur de commande de posture (17), et un état de posture prédéterminé, et donne une instruction au dispositif d'entraînement (60) de faire tourner la portion de pointe (11) dans la direction axiale sur la base d'un résultat de calcul de la force de rotation.

5. Système d'endoscope (1) selon la revendication 4, dans lequel
le dispositif de calcul (30) calcule en outre, sur la base de la différence, une direction dans laquelle la portion de pointe (11) est tournée dans la direction axiale.

6. Système d'endoscope (1) selon la revendication 4, dans lequel
le dispositif de calcul (30) effectue de manière répétée le calcul de la force de rotation et l'instruction donnée au dispositif d'entraînement (60).

7. Système d'endoscope (1) selon la revendication 4, dans lequel
la différence est une valeur comprise dans une plage prédéterminée centrée sur zéro.
